# EUROPÄISCHE PATENTSCHRIFT

(11) **EP 2 181 648 B1**
(45) Veröffentlichungstag und Bekanntmachung des Hinweises auf die Patenterteilung: **12.03.2014**
(21) Anmeldenummer: 09172873.3
(22) Anmeldetag: 13.10.2009
(51) Int. Cl.: A61B 5/0464, A61N 1/362, A61N 1/38

(54) **Einkammer-Herzstimulator**
Single chamber heart simulator
Stimulateur cardiaque à une chambre

(30) Priorität: 04.11.2008 DE 102008043450
(43) Veröffentlichungstag der Anmeldung: 05.05.2010
(73) Patentinhaber: Biotronik CRM Patent AG, 6341 Baar (CH)
(72) Erfinder: Dörr, Thomas, 12437, Berlin (DE); Tietze, Ulrich, 13156, Berlin (DE)
(74) Vertreter: Lindner-Vogt, Karin L.

(56) Entgegenhaltungen:
- WO-A1-99/65570
- WO-A1-2004/105871
- US-A1- 2006 247 703

## Beschreibung

Die Erfindung betrifft einen Einkammer-Herzstimulator, insbesondere einen implantierbaren Herzschrittmacher oder einen implantierbaren Cardioverter/Defibrillator, mit einer rechtsventrikulären Elektrodenleitung. An dieser Elektrodenleitung sind wenigstens eine rechtsventrikuläre Sensingelektrode und eine rechtsventrikuläre Stimulationselektrode angebracht, die im Einzelfall auch von einem einzigen Elektrodenpol gebildet sein können, der dann sowohl als Stimulations- als auch als Sensingelektrode dient.

Unter einem Einkammer-Herzstimulator wird hier ein Herzstimulator verstanden, der nach üblicher Nomenklatur dazu in der Lage ist, nur in einer Herzkammer ein intrakardiales Elektrokardiogramm über eine entsprechende Sensingelektrode aufzunehmen und nur an diese Herzkammer Stimulationsimpulse über eine entsprechende Stimulationselektrode abzugeben. Stimulations- und Sensingelektrode können dabei verschieden voneinander sein oder von demselben Elektrodenpol gebildet sein. Unter einem Einkammer-Herzstimulator soll hier auch ein Herzstimulator verstanden werden, der grundsätzlich auch mit Sensingelektroden in mehreren Herzkammern verbunden werden und so als Mehrkammer-Herzstimulator dienen kann, der aber im Betrieb nur mit einer Elektrodenleitung zum Sensing und zur Stimulation in einer Herzkammer verbunden ist.

Ein Vorteil eines derartigen Einkammer-Herzstimulators ist dessen grundsätzlich einfacher Aufbau gepaart mit der Tatsache, dass im Herzen eines Patienten nur eine einzige Elektrodenleitung implantiert zu werden braucht.

Für die Diskriminierung zwischen ventrikulären (VT) von supraventrikulären Tachykardien (SVT) existieren vielfältige Algorithmen. Für Einkammer-ICDs sind alle die Algorithmen nicht anwendbar, die eine Information des atrialen Rhythmus benötigen. Für diese Einkammer ICDs sind der plötzliche Tachykardie-Onset und die RR-Intervallstabilität etablierte Kriterien zu VT/SVT-Diskriminierung. Des Weiteren werden diese Kriterien durch die Bewertung der Morphologie des QRS-Komplexes ergänzt. Diese morphologie-basierten Algorithmen sind jedoch nur eingeschränkt geeignet, da sich die QRS-Morphologie, abgeleitet in nur einer EKG-Ableitung bei einer VT gegenüber dem Sinusrhythmus unverändert darstellen kann. In diesen Fällen würde eine VT fälschlicherweise als SVT klassifiziert und demzufolge nicht therapiert.

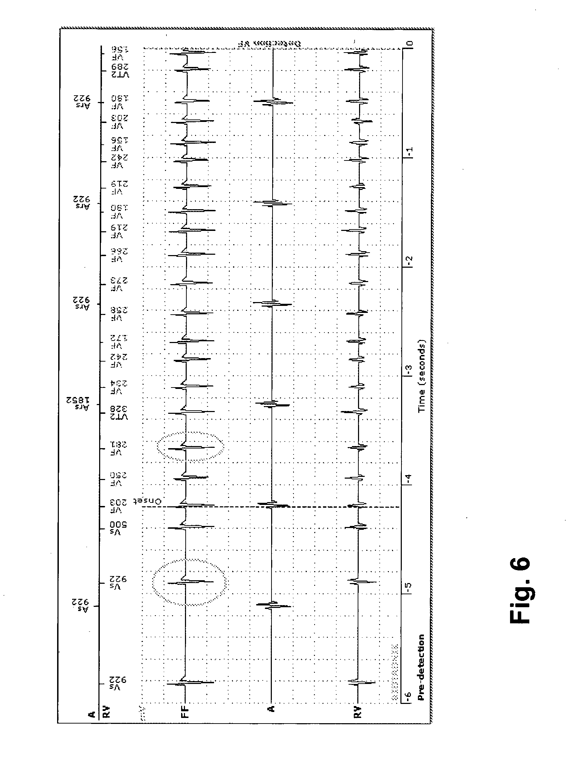

In Figur 6 ist ein solches Beispiel dargestellt. Die Abbildung zeigt das EKG vor und während einer ventrikulären Tachykardie. Im als FF gekennzeichneten Kanal ist die Far Field EKG-Ableitung zwischen der ventrikulären Schockelektrode und dem Gehäuse des ICD dargestellt. Die beiden markierten QRS-Komplexe zeigen die QRS-Morphologie bei Sinusrhythmus und während der ventrikulären Tachykardie. Es ist deutlich zu erkennen, dass die Morphologie identisch ist und somit keinen Anhaltspunkt zur Diskriminierung beinhaltet.

Ein Gerät nach der Präambel von Anspruch 1 ist aus US 2006 247703 bekannt.

Aufgabe der Erfindung ist es, einen Herzstimulator zu schaffen, der eine verbesserte VT/SVT-Diskriminierung ermöglicht.

Erfindungsgemäß wird diese Aufgabe durch einen Herzstimulator nach Anspruch gelöst, wenigstens zum Teil elektrisch leitendes Gehäuse, einer erste Erfassungseinheit zum Erfassen eines ersten Elektrokardiogrammsignals (erster Kanal), einer zweite Erfassungseinheit zum simultanen Erfassen eines zweiten Elektrokardiogrammsignals (zweiter Kanal), eine Morphologie-Analyseeinheit und eine VT/SVT-Diskriminierungseinheit aufweist. Die erste Erfassungseinheit ist über eine ventrikuläre Elektrodenleitung mit wenigstens einer ventrikulären Elektrode und einer weiteren Elektrode verbunden oder zu verbinden und ist ausgebildet, ventrikuläre Herzaktionen vermittels eines über die ventrikuläre Elektrode und die weitere Elektrode ein erstes Elektrokardiogrammsignal aufzunehmen. Die zweite Erfassungseinheit ist einerseits über die ventrikuläre Elektrodenleitung mit wenigstens einer Elektrode dieser ventrikulären Elektrodenleitung verbunden oder zu verbinden und andrerseits mit einer weiteren Elektrode verbunden und ist ausgebildet, über diese Elektroden ein zweites Elektrokardiogrammsignal simultan zum ersten Elektrokardiogrammsignal aufzunehmen, wobei wenigstens eine der beiden Elektroden, über die das zweite Elektrokardiogrammsignal aufzunehmen ist, eine andere Elektrode ist, über die das erste Elektrokardiogrammsignal aufzunehmen ist, so dass die Elektrokardiogrammsignale EKG-Ableitungen darstellen, deren Vektoren ungleich 0° oder 180° sind.

Die Morphologieanalyse-Einheit ist zur Bestimmung von mindestens jeweils einem morphologischen Signalcharakteristikum des ersten Elektrokardiogrammsignals und des simultanen zweiten Elektrokardiogrammsignals in einem zweiten EKG-Kanal ausgebildet. Die VT/SVT-Diskriminierungseinheit ist mit der Morphologieanalyse-Einheit verbunden und ausgebildet, die von der Morphologieanalyse-Einheit bestimmten Signalcharakteristika zur VT/SVT-Diskriminierung zu nutzen.

Die Erfindung geht davon aus, dass sich in zwei EKG-Ableitungen (abgeleitet in nicht parallel verlaufenden Projektionen), bei einer VT mindestens in einer Ableitung eine Änderung der EKG-Morphologie gegenüber Sinusrhythmus darstellt.

Figur 6 zeigt, dass in dem dort abgebildeten rechtsventrikulären IEGM (RV) eine geänderte Signalmorphologie sichtbar ist.

Vorzugsweise ist die erste Erfassungseinheit eine ventrikuläre Sensingeinheit, die über eine ventrikuläre Elektrodenleitung mit wenigstens einem ventrikulären Sensing-Elektrodenpaar verbunden oder zu verbinden ist. Ein solches ventrikuläres Sensing-Elektrodenpaar wird beispielsweise von eine ventrikulären Spitzenelektrode und einer benachbarten Ringelektrode gebildet. Die ventrikuläre Sensingeinheit als erste Erfassungseinheit ist vorzugsweise ausgebildet, das erste Elektrokardiogrammsignal über dieses Elektrodenpaar als bipolares Elektrokardiogrammsignal aufzunehmen und ventrikuläre Herzaktionen in an sich bekannter Weise mittels des bipolar aufgenommen ersten Elektrokardiogrammsignals wahrzunehmen und zu klassifizieren, also ventrikuläre Herzaktionen zu detektieren.

Vorzugsweise ist die zweite Erfassungseinheit einerseits über die ventrikuläre Elektrodenleitung mit wenigstens einer Defibrillationselektrode dieser ventrikulären Elektrodenleitung verbunden oder zu verbinden und andererseits mit dem elektrisch leitenden Gehäuse des Einkammer-Herzstimulators als weitere Elektrode. Eine Defibrillationselektrode ist in der Regel als Schockwendel ausgebildet und besitzt eine im Vergleich zu typischen Stimulations- oder Sensingelektroden wesentlich größere Oberfläche. Die zweite Erfassungseinheit ist vorzugsweise ausgebildet, das zweite Elektrokardiogrammsignal als Farfield-Elektrokardiogrammsignal über diese beiden Elektroden - eine Schockwendel der ventrikulären Elektrodenleitung und das leitende Gehäuse des Stimulators - aufzunehmen.

Alternativ kann die zweite Erfassungseinheit auch über die ventrikuläre Elektrodenleitung mit zwei Defibrillationselektroden (also Schockwendeln) an dieser Elektrodenleitung verbunden oder zu verbinden sein. In diesem Falle ist die zweite Erfassungseinheit dazu ausgebildet, das zweite Elektrokardiogrammsignal als Farfield-Elektrokardiogrammsignal mit diesen beiden Defibrillationselektroden aufzunehmen.

Die VT/SVT-Diskriminierungseinheit ist vorzugsweise ausgebildet, das Verhältnis einander zugeordneter von der Morphologie-Analyseeinheit bestimmter Signalcharakteristika zueinander zu bestimmen und mit wenigstens einem ersten Referenzverhältniswert zu vergleichen. Die Signalcharakteristika können beispielsweise die Maxima eines jeweils detektierten QRS-Komplexes sein. Die Maxima einander entsprechender QRS-Komplexe der beiden parallel aufgenommenen Elektrokardiogrammsignale werden zueinander ins Verhältnis gesetzt und wenigstens mit einem ersten Referenzverhältniswert verglichen.

Vorzugsweise ist der Herzstimulator außerdem eine Zeitmesseinheit, die es erlaubt, die Dauer jeweils aktueller RR-Intervalle bzw. den Kehrwert, also eine jeweils aktuelle ventrikuläre Rate, zu bestimmen und den so bestimmten Wert der VT/SVT-Diskriminierungseinheit zuzuführen. Die VT/SVT-Diskriminierungseinheit ist in diesem Falle dazu ausgebildet, ein jeweiliges RR-Intervall oder die entsprechende ventrikuläre Rate mit wenigstens einem VT-Zonen-Grenzwert zu vergleichen und für den Fall, dass das RR-Intervall kürzer ist als der VT-Zonen-Grenzwert, oder die ventrikuläre Rate größer ist als der VT-Zonen-Grenzwert, ein Verhältnis zu bilden, das sich aus den aktuellen, einander zugeordneten, von der Morphologie-Analyseeinheit bestimmten Signalcharakteristika ergibt und dieses Verhältnis mit dem Referenzverhältniswert zu vergleichen.

Außerdem ist die VT/SVT-Diskriminierungseinheit vorzugsweise dazu ausgebildet, das Verhältnis der einander zugeordneten, von der Morphologie-Analyseeinheit bestimmten Signalcharakteristika, die während eines Normalrhythmus aufgenommen wurden, als Referenzverhältnis zu speichern. Einen Normalrhythmus diktiert die VT/SVT-Diskriminierungseinheit dann, wenn ein jeweiliges RR-Intervall länger oder eine jeweilige ventrikuläre Rate kleiner ist als ein entsprechender VT-Zonen-Grenzwert.

Die Zeitmesseinheit ist vorzugsweise ausgebildet, ein jeweiliges RR-Intervall oder dessen Kehrwert, die ventrikuläre Rate, über mehrere aufeinanderfolgende Herzzyklen zu ermitteln und den jeweils so bestimmten Wert der VT/SVT-Diskriminierungseinheit zuzuführen.

Um eine Unterscheidung nicht nur zwischen Normalrhythmus und Tachykardie zu ermöglichen, sondern auch eine Fibrillation zu detektieren, die sich in der Regel durch noch höhere Herzraten auszeichnet als sie bei einer Tachykardie vorliegen, ist die VT/SVT-Diskriminierungseinheit vorzugsweise dazu ausgebildet, ein jeweiliges RR-Intervall oder die ventrikuläre Rate nicht nur mit einem ersten VT-Zonen-Grenzwert zu vergleichen, sondern auch mit einem zweiten VT-Zonen-Grenzwert. Die beiden VT-Zonen-Grenzwerte bilden dabei eine obere und eine untere Grenze einer Tachykardiezone. Liegt die ventrikuläre Rate oberhalb der oberen Grenze (zweiter VT-Zonen-Grenzwert) der VT-Zone, detektiert die VT/SVT-Diskriminierungseinheit eine ventrikuläre Fibrillation.

Darüber hinaus ist es bevorzugt, wenn die Morphologie-Analyseeinheit dazu ausgebildet ist, ein jeweiliges Signalcharakteristikum einschließlich des zugehörigen Vorzeichens der Signalamplitude des jeweiligen Elektrokardiogrammsignals zu bestimmen und der VT/SVT-Diskriminierungseinheit zuzuführen.

Weiterhin ist die Morphologie-Analyseeinheit vorzugsweise dazu ausgebildet, ein jeweiliges Signalcharakteristikum innerhalb eines Zeitfensters zu bestimmen, das sich über einen Zeitraum erstreckt, welcher um einen vorbestimmten Zeitwert vor einer jeweiligen detektierten ventrikulären Herzaktion beginnt und zu einem Zeitpunkt endet, der um einen bestimmten Zeitwert nach der detektierten ventrikulären Herzaktion liegt. Die Morphologie-Analyseeinheit ist hierzu vorzugsweise mit der ventrikulären Sensingeinheit verbunden, die dazu ausgebildet ist, ventrikuläre Herzaktionen zu detektieren und den Zeitpunkt einer detektierten ventrikulären Herzaktion der Morphologie-Analyseeinheit zuzuführen.

Weitere vorteilhafte Ausgestaltungen der Erfindung ergeben sich aus den Kombinationen der hier beschriebenen vorteilhaften Merkmale sowie der in der nachfolgenden Beschreibung eines Ausführungsbeispiels genannten Merkmale.

Die Erfindung soll nun anhand eines Ausführungsbeispiels mit Bezug auf die Figuren näher erläutert werden. Von diesen zeigt:
- Fig.1:: einen Herzstimulator in Form eines implantierbaren Einkammer-Kardioverter/Defibrillator in Verbindung mit daran angeschlossenen Elektrodenleitungen;
- Fig. 2:: ein schematisches Blockschaltbild des Herzstimulators aus Figur 1;
- Fig. 3:: die für die VT/SVT Diskriminierung vorgesehenen Komponenten des Herzstimulators aus Figuren 1 und 2 in detaillierter Darstellung;
- Fig. 4:: die Arbeitsweise der VT/SVT-Diskriminierungseinheit anhand eines Flussdiagramms;
- Fig.5:: die erfindungsgemäße 2 Kanal-Morphologiebewertung anhand eines Beispiels; und
- Fig. 6:: verschiedene Elektrokardiogramme im Vergleich;

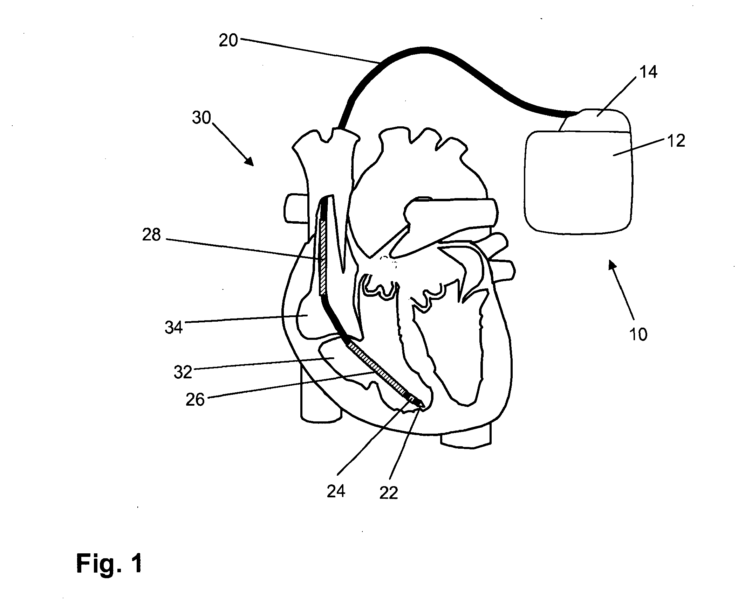

Figur 1 zeigt einen ventrikulären Herzstimulator 10 mit einem Gehäuse 12 und einem Header 14. Das Gehäuse 12 ist hohl und besitzt wenigstens teilweise eine elektrisch leitende Oberfläche, typischer Weise besteht das Gehäuse 12 aus einem biokompatiblen Metall wie Titan. In dem Gehäuse 12 befinden sich eine Batterie und elektronische Komponenten des Herzstimulators 10. Der Header 14 enthält Anschlussbuchsen, beispielsweise für eine Elektrodenleitung.

In Figur 1 ist der Herzstimulator 10 mit einer flexiblen, implantierbaren Elektrodenleitung 20 verbunden.

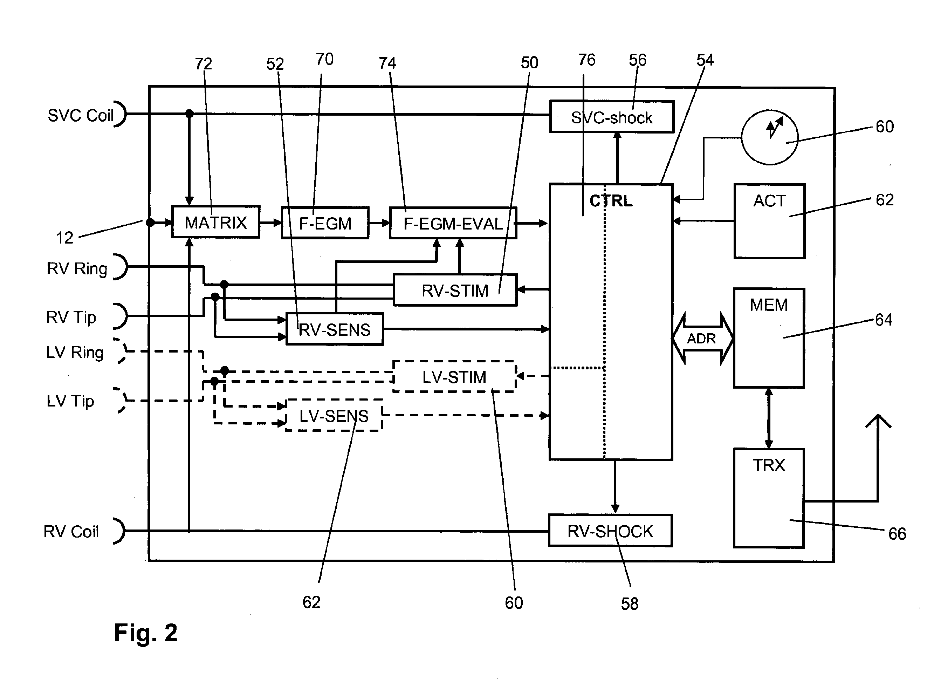

Die abgebildete Elektrodenleitung 20 ist eine ventrikuläre Elektrodenleitung, die an ihrem distalen Ende eine ventrikuläre Tip-Elektrode 22 und eine ventrikulären Ringelektrode 24 aufweist, die zusammen einen bipolare Wahrnehmungs- und Stimulationspol bilden. Von diesen dient die ventrikuläre Tip-Elektrode 22 als Stimulationselektrode. Die ventrikuläre Tip-Elektrode 22 und die ventrikuläre Ringelektrode 24 bilden zusammen ein Elektrodenpaar für das bipolare Sensing ventrikulärer Ereignisse. Hierzu sind die ventrikuläre Tip-Elektrode 22 und die ventrikuläre Ringelektrode 24 mit einer ventrikulären Stimulationseinheit und einer ventrikulären Sensingeinheit im Inneren des Gehäuses 12 des Herzstimulators 10 verbunden (Näheres ist mit Bezug auf Fig. 2 beschrieben). Außerdem besitzt die ventrikuläre Elektrodenleitung 20 eine ventrikuläre Schockwendel 26 als distale Schockwendel und eine proximale Schockwendel 28. Die ventrikuläre Schockwendel 26 ist dabei so auf der ventrikulären Elektrodenleitung 20 angeordnet, dass sie dann, wenn sie in ein Herz 30 eingeführt ist, im rechten Ventrikel 32 des Herzens angeordnet ist. Die ventrikuläre Tip-Elektrode 22 befindet sich dann im Apex des Ventrikels 32 des Herzens 30. Die proximale Schockwendel 28 befindet sich bei implantierter ventrikulärer Elektrodenleitung 20 in der Vena cava superior des Herzens 30. Eine spezielle Elektrodenleitung zur Stimulation des rechten Atriums 34 des Herzens 30 ist nicht vorgesehen. Ebenso gibt es keine atriale Sensingelektrode. Der Herzstimulator 10 kommt ganz ohne atriale Elektrodenleitung aus und bietet dennoch die Funktionalität solcher Herzstimulatoren, die mit einer atrialen Elektrodenleitung verbunden sind. Dies wird anschließend näher erläutert.

Figur 2 zeigt in einem schematischen Blockschaltbild Komponenten des Herzstimulators 10, die im Inneren des Gehäuses 12 angeordnet sind. Dabei ist die Darstellung nicht notwendiger Weise abschließend. Insbesondere sind in Fig. 2 Komponenten gestrichelt dargestellt, die bei einem Herzstimulator 10 aus Fig. 1 nicht verwirklicht sind.

Wie bereits angedeutet besitzt der Herzstimulator 10 in seinem Header 14 Anschlusskontakte für den Anschluss entsprechender Gegenkontakte eines Elektrodenleitungssteckers am proximalen Ende der Elektrodenleitung 20. Diese Kontakte dienen zur elektrischen Verbindung mit den Elektroden der Elektrodenleitung 20. So ist die proximale Schockwendel 38 mit dem Kontakt SVC Coil verbunden, die ventrikuläre (distale) Schockwendel 26 mit dem Anschluss RV Coil, die rechtsventrikuläre Tip-Elektrode 22 mit dem Anschluss RV Tip und die rechtsventrikuläre Ringelektrode mit dem Anschluss RV Ring. Über die Anschlüsse RV Tip und RV Ring sind die rechtsventrikuläre Tip-Elektrode 22 und die rechtsventrikuläre Ringelektrode 24 jeweils mit einer rechtsventrikulären Stimulationseinheit 50 und einer rechtsventrikulären Sensingeinheit 52 verbunden. Die rechtsventrikuläre Sensingeinheit 52 ist dabei ausgangsseitig mit einer Stimulationssteuereinheit 54 verbunden, die ihrerseits wiederum einen Ausgang besitzt, der mit der rechtsventrikulären Stimulationseinheit 50 verbunden ist. Die rechtsventrikuläre Stimulationseinheit 50 ist dazu ausgebildet, auf ein entsprechendes Steuersignal der Stimulationssteuereinheit 54 hin einen ventrikulären Stimulationsimpuls zu generieren und wenigstens über den Kontakt RV Tip abzugeben.

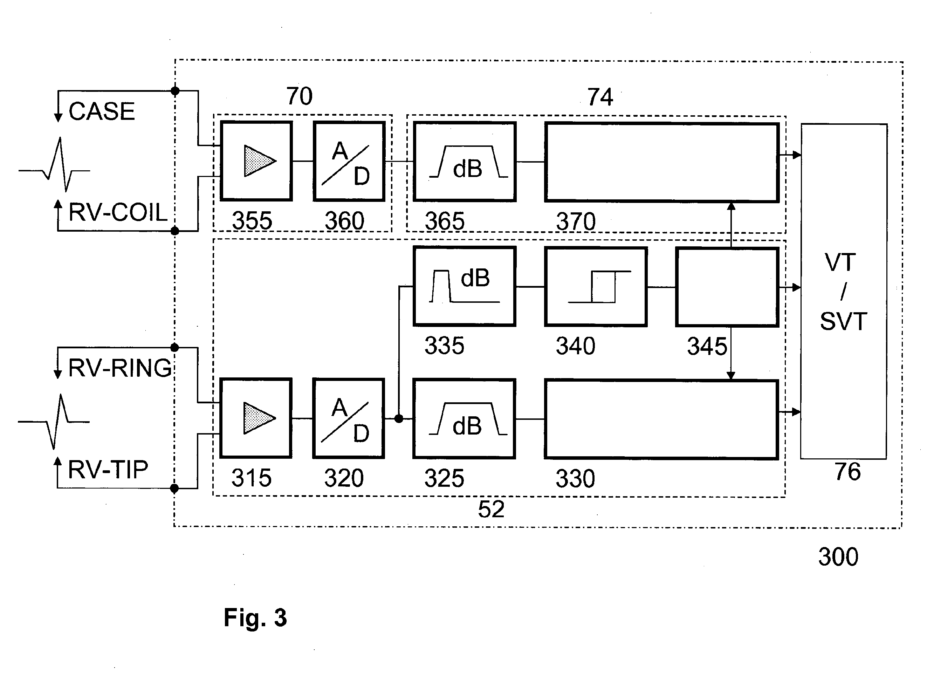

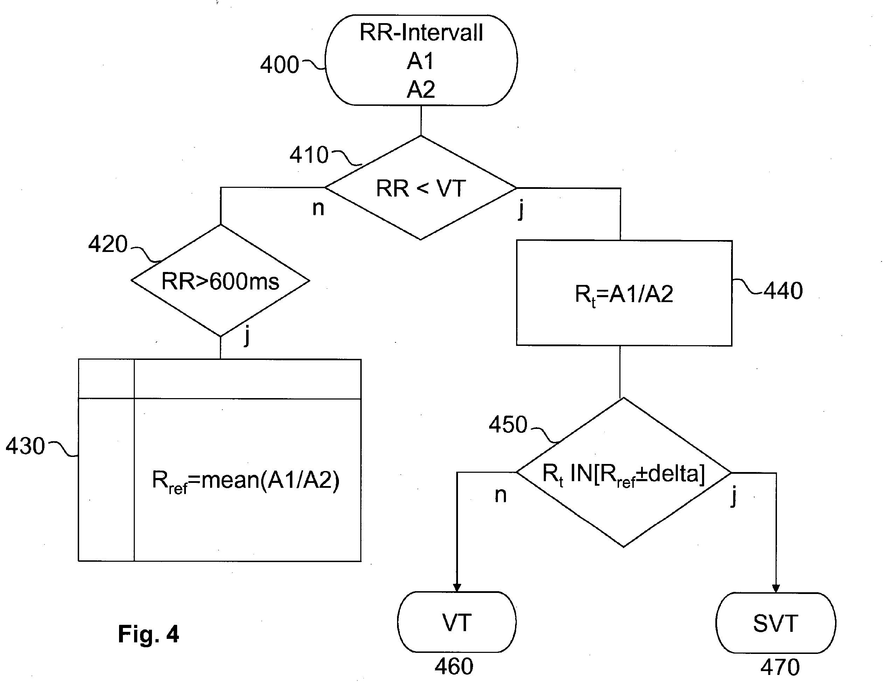

Die Funktionsweise der rechtsventrikulären Sensingeinheit 52 wird nachfolgend mit Bezug auf Figuren 3 und 4 noch näher erläutert. Die rechtsventrikuläre Sensingeinheit ist grundsätzlich dazu ausgebildet, den Verlauf eines Signals auszuwerten, der sich aus der Differenz der an den Anschlüssen RV Tip und RV Ring anliegenden Potenziale ergibt. Dieser Signalverlauf enthält typischer Weise Signalspitzen, die im Falle ventrikulärer Depolarisationen auftreten. Ventrikuläre Depolarisationen gehen einer Kontraktion des ventrikulären Myokards voraus und kennzeichnen somit ventrikuläre Senseereignisse. Diese können aus dem Signalverlauf detektiert werden, indem die Potenziale mit einem Schwellwert verglichen werden. Dieser ist so eingestellt, dass die mit ventrikulären Depolarisationen einhergehenden Signalspitzen den Schwellwert überschreiten, so dass die ventrikuläre Sensingeinheit 52 ventrikuläre Senseereignisse durch Schwellwertvergleich bei Schwellwertüberschreitung detektieren kann.

Zur Erzeugung und Abgabe von Defibrillationsschocks sind außerdem Schockgeneratoren 56 und 58 vorgesehen, die über den Anschluss SVC Coil mit der proximalen Schockwendel 28 bzw. über den Anschluss RV Coil mit der distalen Schockwendel 26 verbunden sind. Die beiden Defibrillationsschock-Generatoren 56 und 58 sind dabei jeweils ebenfalls mit der Stimulationssteuereinheit 54 verbunden. Weitere Merkmale des Herzstimulators 10 sind ein Zeitgeber 60, der beispielsweise zur Intervallmessung und Herzratenbestimmung herangezogen wird, sowie ein Aktivitätssensor 62, der ausgebildet ist, eine physische Aktivität eines Patienten, beispielsweise durch Bewegungserfassung, zu erfassen, um es der Stimulationssteuereinheit 54 zu ermöglichen, eine Stimulationsrate an den physiologischen Bedarf eines Patienten anzupassen. Weiterhin besitzt der Herzstimulator 10 einen Speicher 64 zum Speichern von Steuerparametern sowie von physiologischen Parametern, die beispielsweise durch Auswerten der verschiedenen vom Herzstimulator 10 aufgenommenen Signale gewonnen werden.

Schließlich besitzt der Herzstimulator 10 auch eine Telemetrieeinheit 66, über die der Herzstimulator 10 gewonnene und gespeicherte physiologische Parameter an ein externes Gerät drahtlos übermitteln kann oder aber über die der Herzstimulator 10 Steuerparameter empfangen kann, die die Funktionsweise des Herzstimulators 10 steuern.

Im Sinne der Erfindung besitzt der Herzstimulator 10 außerdem eine Fernfeldelektrokardiogramm-Erfassungseinheit 70, die eingangsseitig einerseits mit einer Schaltmatrix 72 verbunden ist, über die die Fernfeldelektrokardiogramm-Erfassungseinheit 70 wahlweise mit verschiedenen Elektrodenpolen zu verbinden ist.

Grundsätzlich ergeben sich die folgenden Elektrodenkonfigurationen für das Eingangssignal der Fernfeldelektrokardiogramm-Erfassungseinheit 70:

| | erstes EKG | zweites EKG |
|---|---|---|
| Variante A: | RV-Coil - Gehäuse | SVC-Coil - Gehäuse |
| Variante B: | RV-Coil - Gehäuse | RV-Coil - SVC-Coil |
| Variante C: | SVC Coil - Gehäuse | RV-Coil - SVC-Coil |
| Variante D: | RV Tip - RV Ring | RV-Coil - Gehäuse |

In allen dieser Kombinationen ergeben sich EKG-Vektoren, die nicht parallel zueinander verlaufen.

Die Variante D ist jedoch zu bevorzugen, da diese keine "Dual Coil"-Elektrode als Voraussetzung für die VT/SVT-Diskriminierung aufweist. Die folgenden Implementierungsbeispiele beschränken sich daher auf diese bevorzugte Kombination

Das mehrere Elektrodenkonfigurationen für die Bestimmung der EKG-Ableitungen möglich sind, ist in dem Ausführungsbeispiel die Schaltmatrix 72 vorgesehen, die entweder manuell durch den Anwender programmierbar, oder aber automatisch anhand von Elektrodenimpedanzen und Signalqualität die jeweils günstigste Ableitung auswählt.

Aus der Potenzialdifferenz zwischen den beiden ausgewählten Eingängen ergibt sich ein Fernfeldelektrokardiogramm, das von der Fernfeldelektrokardiogramm-Erfassungseinheit 70 aufgenommen, verstärkt, analog-digital-gewandelt und gefiltert wird, wie dies mit Bezug auf Fig. 3 noch näher erläutert wird. Das so gewonnene und aufbereitete Fernfeldelektrokardiogramm liegt an einem Ausgang der Fernfeldelektrokardiogramm-Erfassungseinheit 70 an. Dieser Ausgang ist mit einem Eingang einer Fernfeldelektrokardiogramm-Auswerteeinheit 74 verbunden. Diese Fernfeldelektrokardiogramm-Auswerteeinheit 74 besitzt außerdem noch einen Eingang, der mit der rechtsventrikulären Sensingeinheit 52 sowie einen weiteren Eingang, der mit der rechtsventrikulären Stimulationseinheit 50 verbunden ist. Alternativ dazu kann auch ein einziger Eingang der Fernfeldelektrokardiogramm-Auswerteeinheit 74 vorgesehen sein, der mit der Stimulationssteuereinheit 54 verbunden ist. Diese weiteren Eingänge der Fernfeldelektrokardiogramm-Auswerteeinheit 74 dienen dazu, der Fernfeldelektrokardiogramm-Auswerteeinheit 74 Signale zuzuführen, die ventrikuläre Sensingereignisse bzw. ventrikuläre Stimulationsereignisse charakterisieren. Die Fernfeldelektrokardiogramm-Auswerteeinheit 74 ist ausgebildet, das seitens der Fernfeldelektrokardiogramm-Erfassungseinheit 70 gebildete Fernfeldelektrokardiogramm unter Berücksichtigung ventrikulärer Sensingereignisse und Stimulationsereignisse charakterisierender Signale auszuwerten, um in dem Fernfeldelektrokardiogramm Signalmerkmale zu detektieren, die atriale (Sense-)Ereignisse charakterisieren.

Die Fernfeldelektrokardiogramm-Auswerteeinheit 74 ist ausgangsseitig mit einer VT/SVT-Diskriminierungseinheit 76 als Teil der Stimulationssteuereinheit 54 verbunden. Die VT/SVT-Diskriminierungseinheit 76 enthält eine nicht näher dargestellte Morphologie-Analyseeinheit, die ausgebildet ist, wenigstens ein bestimmtes Signalcharakteristikum wie beispielsweise die Maximalamplitude eines jeweiligen QRS-Komplexes der von der ventrikulären Sensingeinheit 52 sowie der Fernfeldelektrokardiogramm-Erfassungseinheit 70 aufgenommenen Elektrokardiogramme zu bestimmen. Dies ist weiter unter näher erläutert.

Figur 3 zeigt ein Blockschaltbild mit denjenigen Komponenten eines Einkammer-ICD als Herzstimulator dargestellt, die eine erfindungsgemäße VT/SVT-Diskriminierung bewirken.

Der ICD ist über die Anschlüsse RV-TIP und RV-RING mit der bipolaren Wahrnehmungs- und Stimulationselektrodenleitung 20 verbunden. Das mittels Elektroden 22 und 24 dieser Elektrodenleitung 20 abgeleitete intrakardiale Elektrogramm (IEGM) wird zunächst durch einen Verstärker 315 verstärkt und mittels eines A-D-Umsetzers 320 digitalisiert. Anschließend wird das so gewonnene Digitalsignal einer schmalbandigen Filterstufe 335 zugeführt. Diese schmalbandige Filterstufe 335, z.B. ein Bandpass 2. Ordnung von 20 bis 40Hz, dient der Filterung des IEGM für die ventrikuläre Wahnehmungsfunktion, wie in einem ICD üblich. Anschließend erfolgt in einer adaptiven Komparatorstufe 340 die Wahrnehmung (Detektion) der ventrikulären Ereignisse (Sensing), z.B. durch SchwellwertVergleich. In einer nachfolgenden Zeitmesseinheit 345 werden die zeitlichen Abstände zwischen aufeinander folgenden ventrikulären Ereignissen (RR-Intervalle) bestimmt und klassifiziert, also z.B. verschiednen Tachykardiezonen zugeordnet.

Parallel zu der üblichen Wahrnehmungsfunktion des ventrikulären IEGMs wird das digitalisierte IEGM-Signal einer zweiten, parallel angeordneten breitbandigen Filterstufe 325 zugeführt. Die hier verwendete Filtercharakteristik ist optimiert, um einen möglichst großen spektralen Anteil der QRS-Morphologie zu erhalten, Störsignale aber weitgehend zu eliminieren. An diese breitbandige Filterstufe 325 schließt sich eine Einheit 330 zur Bestimmung der maximalen Amplitude des QRS-Komplexes an. Diese Einheit 330 enthält einen Ringspeicher, in dem das IEGM für eine Zeit von 50ms bis 250ms zwischengespeichert werden kann. Die Bestimmung des QRS-Maximums erfolgt in dieser Einheit 330 immer getriggert auf ein ventrikuläres Ereignis. Dieser Trigger wird von der Zeitmesseinheit 345 zur Verfügung gestellt. Wird die Einheit 330 getriggert, so bestimmt diese vorzeichenbehaftet die maximale IEGM-Amplitude im zeitlichen Umfeld des Triggerzeitpunktes, z.B. 100ms vor bis 100ms nach dem Triggerzeitpunkt.

Die so bestimmte QRS-Maximalamplitude des ersten Elektrokardiogrammsignals wird der VT/SVT-Diskriminierungseinheit 76 zur Verfügung gestellt.

Eine weitere EKG-Ableitung erfolgt zwischen distaler Schockwendel 26 über den Anschluss RV-SHOCK und dem Gehäuse 12 des ICD (über den Anschluss CASE). Ein so gewonnenes Far-Field-Elektrokardiogramm als zweitem Elektrokardiogrammsignal wird ebenfalls durch einen Verstärker 355 verstärkt und mittels eines zweiten A-D-Umsetzers 360 digitalisiert, mittels einer Filterstufe 365 breitbandig gefiltert und einer weiteren Einheit 370 zur Bestimmung des QRS-Maximums zugeführt. Die Bestimmung des QRS-Maximums erfolgt analog zum ersten Kanal (durch die Einheit 330). Die so im zweiten Elektrokardiogrammsignal bestimmten Maximalwerte werden ebenfalls an die VT/SVT-Diskriminierungseinheit 76 weitergegeben.

Der VT/SVT-Diskriminierungseinheit 76 werden außerdem die durch die Zeitmesseinheit 345 klassifizierten RR-Intervalle zugeführt.

Figur 4 illustriert die Arbeitsweise der VT/SVT-Diskriminierungseinheit 76 aus Figur 3. Der VT/SVT-Diskriminierungseinheit 76 werden mit jedem ventrikulären Ereignis das aktuelle RR-Intervall und das QRS-Signalmaximum des ersten Elektrokardiogrammsignals (A1) und des zweiten Elektrokardiogrammsignals (A2) zu Verfügung gestellt (400).

Zunächst wird geprüft, ob das aktuelle RR-Intervall innerhalb einer VT-Zone liegt (410). Ist dies nicht der Fall, wird geprüft, ob das RR-Intervall größer als 600ms ist (420). Ist dies der Fall, dann berechnet die VT/SVT-Diskriminierungseinheit 76 einen Referenzwert für das Verhältnis der QRS-Maximalamplituden Rref (430), wobei dieser Referenzwert typischerweise den Median mehrerer aufeinander folgend gemessener QRS-Komplexe entspricht.

Liegt das aktuelle RR-Intervall jedoch innerhalb einer VT-Zone (410), dann wird ein Verhältnis der QRS-Maximalamplituden für das aktuelle Intervall berechnet (440). Anschließend wird geprüft, ob dieser Wert (Rt) dem Referenzwert (Rref) zuzüglich einer Toleranz (delta) entspricht. Ist dies der Fall, dann wird das aktuelle RR-Intervall als eine SVT gezählt (470), andernfalls als VT (460).

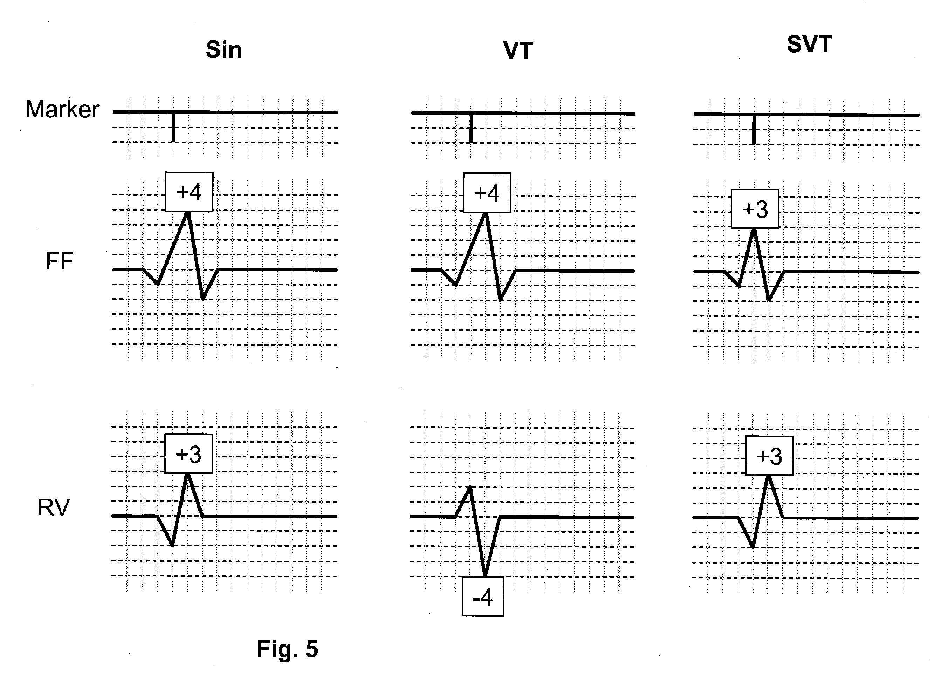

In Figur 5 ist die 2 Kanal-Morphologiebewertung im Beispiel dargestellt. Die Zeile Marker zeigt den Zeitpunkt des wahrgenommenen ventrikulären Ereignisses an und damit den Trigger zur Morphologieanalyse an. Die Zeile FF zeigt die jeweilige Signalmorphologie im Far-Field-Kanal und die Zeile RV zeigt die Morphologie im bipolaren rechtsventrikulären IEGM-Kanal an.

Die Spalte *Sin* kennzeichnet ein Beispiel mit Sinusrhythmus. Die Spalte *VT* zeigt dass Beispiel einer Tachykardie, die nur in einem der beiden Kanäle eine Morphologieänderung aufweist (ähnlich wie in Abbildung 1) und die Spalte *SVT* zeigt das Beispiel einer supraventrikulären Tachykardie.

Die VT/SVT-Diskriminierungseinheit 76 bestimmt den Referenzwert Rref bei Sinusrhythmus. Im Beispiel Rref= +4 / +3 = 1,33.

Wird eine VT wahrgenommen, so ergibt die Ereignisbewertung einen Wert Rt= +4 / -4 = -1 (minus 1).

Bei einer Toleranz von delta=0,5 liegt Rt außerhalb von [Rref-delta, Rref+delta] und wird damit als VT gezählt.

Wird eine SVT wahrgenommen, so ergibt die Ereignisbewertung einen Wert Rt= +3 / +3 = 1 (plus 1).

Bei einer Toleranz delta=0,5 liegt Rt außerhalb von [Rref-delta, Rref+delta] und wird damit als SVT gezählt.

Neben der maximalen Signalamplitude sind weitere Morphologiekriterien für die im Ausführungsbeispiel beschriebe Diskriminierung entweder einzeln oder in Kombination anwendbar (z.B. QRS-Breite, Integral der Fläche unter dem QRS-Komplex etc.).

## Patentansprüche

1. Einkammer-Herzstimulator (10) mit
- einem wenigstens zum Teil elektrisch leitenden Gehäuse (12),
- einer ersten Erfassungseinheit (52), die über eine ventrikuläre Elektrodenleitung mit wenigstens einer ventrikulären Elektrode und einer weiteren Elektrode verbunden oder zu verbinden ist und die ausgebildet ist, ventrikuläre Herzaktionen vermittels eines über die ventrikuläre Elektrode und die weitere Elektrode ein erstes Elektrokardiogrammsignal aufzunehmen,
- einer zweiten Erfassungseinheit (70), die einerseits über die ventrikuläre E-lektrodenleitung mit wenigstens einer Elektrode dieser ventrikulären Elektrodenleitung verbunden oder zu verbinden ist und andrerseits mit einer weiteren Elektrode verbunden und die ausgebildet ist, über diese Elektroden ein zweites Elektrokardiogrammsignal simultan zum ersten Elektrokardiogrammsignal aufzunehmen, wobei wenigstens eine der beiden Elektroden, über die das zweite Elektrokardiogrammsignal aufizunehmen ist, eine andere Elektrode ist, über die das erste Elektrokardiogrammsignal aufzunehmen ist, so dass die Elektrokardiogrammsignale EKG-Ableitungen darstellen, deren Vektoren ungleich 0° oder 180° sind,
- einer Morphologieanalyseeinheit (76), die mit der ersten und der zweiten Erfassungseinheit verbunden und ausgebildet ist, mindestens jeweils ein morphologisches Signalcharakteristikum des ersten Elektrokardiogrammsignals und des simultanen zweiten Elektrokardiogrammsignals zu bestimmen, und
- einer VT/SVT-Diskriminierungseinheit (76), die mit der Morphologieanalyse-Einheit verbunden oder diese umfasst und die ausgebildet ist, die von der Morphologieanalyse-Einheit bestimmten Signalcharakteristika zur VT/SVT-Diskriminierung zu nutzen
**dadurch gekennzeichnet,**
**dass** die VT/SVT-Diskriminierungseinheit ausgebildet ist, das Verhältnis der von der Morphologieanalyse-Einheit bestimmten Signalcharakteristika zueinander zu bestimmen und mit wenigstens einem ersten Referenzverhältniswert zu vergleichen.

2. Herzstimulator nach Anspruch 1, **dadurch gekennzeichnet, dass** die erste Erfassungseinheit eine ventrikuläre Sensingeinheit ist, die über eine ventrikuläre Elektrodenleitung mit wenigstens einem ventrikulären Sensing-Elektrodenpaar verbunden oder zu verbinden ist und die ausgebildet ist, das erste Elektrokardiogrammsignal über dieses Elektrodenpaar als bipolares Elektrokardiogrammsignal aufzunehmen und ventrikuläre Herzaktionen vermittels des bipolar aufgenommenen ersten Elektrokardiogrammsignals wahrzunehmen und zu klassifizieren (detektieren)

3. Herzstimulator nach Anspruch 1 oder 2, **dadurch gekennzeichnet, dass** die zweite Erfassungseinheit einerseits über die ventrikuläre Elektrodenleitung mit wenigstens einer Defibrillations-Elektrode (Schockwendel) dieser ventrikulären Elektrodenleitung verbunden oder zu verbinden und andrerseits mit dem elektrisch leitenden Gehäuse (12) des Einkammer-Herzstimulators als weiterer Elektrode verbunden und die ausgebildet ist, das zweite Elektrokardiogrammsignal als Far-Field-Elektrokardiogrammsignal über diese beiden Elektroden aufzunehmen.

4. Herzstimulator nach Anspruch 1 oder 2, **dadurch gekennzeichnet, dass** die zweite Erfassungseinheit über die ventrikuläre Elektrodenleitung mit zwei Defibrillations-Elektroden (Schockwendeln) dieser ventrikulären Elektrodenleitung verbunden oder zu verbinden und ausgebildet ist, das zweite Elektrokardiogrammsignal als Far-Field-Elektrokardiogrammsignal über diese beiden Defibrillations-Elektroden aufzunehmen.

5. Herzstimulator nach einem der Ansprüche 1 bis 4, **dadurch gekennzeichnet, dass** die VT/SVT-Diskriminierungseinheit ausgebildet ist, das Verhältnis der einander zugeordneten, von der Morphologieanalyse-Einheit bestimmten Signalcharakteristika mit einem Toleranzbereich um den ersten Referenzverhältniswert herum zu vergleichen und bei überschreiten des Toleranzbereichs eine ventrikuläre Tachykardie (VT) zu detektieren und bei Unterschreiten des Toleranzbereichs eine supraventrikuläre Tachykardie (SVT) oder umgekehrt.

6. Herzstimulator nach Anspruch 5, **dadurch gekennzeichnet,**
- **dass** der Herzstimulator eine Zeitmesseinheit (345) aufweist, die ausgebildet ist, den zeitlichen Abstand aufeinander folgender ventrikulärer Herzaktionen als jeweiliges RR-Intervall oder dessen Kehrwert als jeweilige ventrikuläre Rate zu bestimmen und den so bestimmten Wert der VT/SVT-Diskriminierungseinheit (76) zuzuführen, und
- **dass** die VT/SVT-Diskriminierungseinheit (76) ausgebildet ist, ein jeweiliges RR-Intervall oder eine jeweilige ventrikuläre Rate mit wenigstens einem VT-Zonen-Grenzwert zu vergleichen und für den Fall, dass ein jeweiliges RR-Intervall kürzer ist, als der entsprechende VT-Zonen-Grenzwert oder eine jeweilige ventrikuläre Rate größer ist, als der entsprechende VT-Zonen-Grenzwert, ein jeweiliges Verhältnis der aktuellen, einander zugeordneten, von der Morphologieanalyse-Einheit bestimmten Signalcharakteristika zueinander zu bestimmen und mit wenigstens dem ersten Referenzverhältniswert zu vergleichen.

7. Herzstimulator nach Anspruch 6, **dadurch gekennzeichnet, dass** die VT/SVT-Diskriminierungseinheit (76) ausgebildet ist, ein Verhältnis der einander zugeordneten, von der Morphologieanalyse-Einheit bestimmten Signalcharakteristika für den Fall, dass ein jeweils zugehöriges RR-Intervall länger ist, als der entsprechende VT-Zonen-Grenzwert oder eine jeweils zugehörige ventrikuläre Rate kleiner ist, als der entsprechende VT-Zonen-Grenzwert als Referenzverhältniswert zu speichern.

8. Herzstimulator nach Anspruch 6 oder 7, **dadurch gekennzeichnet, dass** die Zeitmesseinheit (345) ausgebildet ist, ein jeweiliges RR-Intervall oder dessen Kehrwert als jeweilige ventrikuläre Rate über mehrere aufeinander folgende Herzzyklen zu mitteln und ein jeweils so bestimmtes jeweiliges RR-Intervall oder dessen Kehrwert als jeweilige ventrikuläre Rate der VT/SVT-Diskriminierungseinheit (76) zuzuführen.

9. Herzstimulator nach einem der Ansprüche 5 bis 8, **dadurch gekennzeichnet, dass** die VT/SVT-Diskriminierungseinheit ausgebildet ist, ein jeweiliges RR-Intervall oder eine jeweilige ventrikuläre Rate mit wenigstens zwei VT-Zonen-Grenzwert zu vergleichen und das jeweilige RR-Intervall oder die jeweilige ventrikuläre Rate einer VT-Zone zuzuordnen, wenn das jeweilige RR-Intervall kürzer ist, als ein erster VT-Zonengrenzwert und länger als ein zweiter VT-Zonen-Grenzwert bzw. die jeweilige ventrikuläre Rate größer als ein erster VT-Zonengrenzwert und kleiner als ein zweiter VT-Zonen-Grenzwert ist, und das jeweilige RR-Intervall oder die jeweilige ventrikuläre Rate einer VF-Zone zuzuordnen, wenn das jeweilige RR-Intervall kürzer ist, als der erste und der zweite VT-Zonen-Grenzwert bzw. die jeweilige ventrikuläre Rate größer als der erste und der zweite VT-Zonen-Grenzwert ist.

10. Herzstimulator nach einem der Ansprüche 1 bis 9, **dadurch gekennzeichnet, dass** die Morphologie-Analyseeinheit dazu ausgebildet ist, ein jeweiliges Signalcharakteristikum einschließlich des zugehörigen Vorzeichens der Signalamplitude des jeweiligen Elektrokardiogrammsignals zu bestimmen und der VT/SVT-Diskriminierungseinheit zuzuführen.

11. Herzstimulator nach einem der Ansprüche 1 bis 10, **dadurch gekennzeichnet, dass** die Morphologie-Analyseeinheit dazu ausgebildet ist, ein jeweiliges Signalcharakteristikum innerhalb eines Zeitfensters zu bestimmen, das sich über einen Zeitraum erstreckt, welcher eine vorbestimmte Zeitdauer vor einer jeweiligen detektierten ventrikulären Herzaktion beginnt und zu einem Zeitpunkt endet, der um eine vorbestimmte Zeitdauer nach der detektierten ventrikulären Herzaktion liegt.

12. Herzstimulator nach einem der Ansprüche 2 bis 11, **dadurch gekennzeichnet, dass** die Morphologie-Analyseeinheit mit der ventrikulären Sensingeinheit verbunden ist, die dazu ausgebildet ist, ventrikuläre Herzaktionen zu detektieren und den Zeitpunkt einer detektierten ventrikulären Herzaktion der Morphologie-Analyseeinheit zuzuführen

## Claims

1. A single-chamber cardiac stimulator (10), comprising
- an at least partially electrically conductive housing (12),
- a first detection unit (52), which is connected or is to be connected via a ventricular electrode line to at least one ventricular electrode and one further electrode and is designed to record ventricular cardiac activity by means of a via the ventricular electrode and the further electrode a first electrocardiogram signal,
- a second detection unit (70), which on the one hand is connected or is to be connected via the ventricular electrode line to at least one electrode of this ventricular electrode line and on the other hand is connected to a further electrode and is designed to record a second electrocardiogram signal simultaneously with the first electrocardiogram signal via these electrodes, wherein at least one of the two electrodes, via which the second electrocardiogram signal is to be recorded, is another electrode via which the first electrocardiogram signal is to be recorded, such that the electrocardiogram signals constitute ECG leads of which the vectors are not equal to 0° or 180°,
- a morphology analysis unit (76), which is connected to the first and the second detection unit and is designed to determine at least one morphological signal characteristic of the first electrocardiogram signal and of the simultaneous second electrocardiogram signal, and
- a VT/SVT discrimination unit (76), which is connected to or comprises the morphology analysis unit and is designed to utilise the signal characteristics determined by the morphology analysis unit for VT/SVT discrimination,
**characterised in that**
the VT/SVT discrimination unit is designed to determine the ratio of the signal characteristics determined by the morphology analysis unit to one another and to compare this ratio with at least one first reference ratio value.

2. The cardiac stimulator according to Claim 1, **characterised in that** the first detection unit is a ventricular sensing unit, which is connected or is to be connected via a ventricular electrode line to at least one ventricular sensing electrode pair and is designed to record the first electrocardiogram signal via this electrode pair as a bipolar electrocardiogram signal and to sense and to classify (detect) ventricular cardiac activity via the first electrocardiogram signal, recorded by bipolar leads.

3. The cardiac stimulator according to Claim 1 or 2, **characterised in that** the second detection unit on the one hand is connected or is to be connected via the ventricular electrode line to at least one defibrillation electrode (shock coil) of this ventricular electrode line and on the other hand is connected to the electrically conductive housing (12) of the single-chamber cardiac stimulator as a further electrode and is designed to record the second electrocardiogram signal as a far-field electrocardiogram signal via these two electrodes.

4. The cardiac stimulator according to Claim 1 or 2, **characterised in that** the second detection unit is connected or is to be connected via the ventricular electrode line to two defibrillation electrodes (shock coils) of this ventricular electrode line and is designed to record the second electrocardiogram signal as a far-field electrocardiogram signal via these two defibrillation electrodes.

5. The cardiac stimulator according to one of Claims 1 to 4, **characterised in that** the VT/SVT discrimination unit is designed to compare the ratio of the mutually assigned signal characteristics determined by the morphology analysis unit with a tolerance range around the first reference ratio value, and, if this ratio exceeds the tolerance range, to detect a ventricular tachycardia (VT), and, if this ratio is below the tolerance range, to detect a supraventricular tachycardia (SVT) or vice versa.

6. The cardiac stimulator according to Claim 5, **characterised in that**
- the cardiac stimulator has a time measuring unit (345) which is designed to determine the period of time between successive ventricular cardiac activity as a respective RR interval or its inverse as a respective ventricular rate and to send the value thereby determined to the VT/SVT discrimination unit (76), and
- the VT/SVT discrimination unit (76) is designed to compare a respective RR interval or a respective ventricular rate with at least one VT zone limit value, and, in the event that a respective RR interval is shorter than the corresponding VT zone limit value or a respective ventricular rate is larger than the corresponding VT zone limit value, to determine a respective ratio of the current mutually assigned signal characteristics determined by the morphology analysis unit to one another and to compare this ratio with at least the first reference ratio value.

7. The cardiac stimulator according to Claim 6, **characterised in that** the VT/SVT discrimination unit (76) is designed to save as the reference ratio value a ratio of the mutually assigned signal characteristics determined by the morphology analysis unit for the case when a respective RR interval is longer than the corresponding VT zone limit value or a respective ventricular rate is lower than the corresponding VT zone limit value.

8. The cardiac stimulator according to Claim 6 or 7, **characterised in that** the time measuring unit (345) is designed to average a respective RR interval or its inverse as the respective ventricular rate over several successive cardiac cycles and to send a respective RR interval thereby determined or its inverse as the respective ventricular rate to the VT/SVT discrimination unit (76).

9. The cardiac stimulator according to one of Claims 5 to 8, **characterised in that** the VT/SVT discrimination unit is designed to compare a respective RR interval or a respective ventricular rate with at least two VT zone limit values and to assign the respective RR interval or the respective ventricular rate to a VT zone when the respective RR interval is shorter than a first VT zone limit value and is longer than a second VT zone limit value and/or the respective ventricular rate is higher than a first VT zone limit value and is lower than a second VT zone limit value, and to assign the respective RR interval or the respective ventricular rate to a VF zone when the respective RR interval is shorter than the first and second VT zone limit values and/or the respective ventricular rate is greater than the first and second VT zone limit values.

10. The cardiac stimulator according to one of Claims 1 to 9, **characterised in that** the morphology analysis unit is designed to determine a respective signal characteristic including the respective plus or minus sign of the signal amplitude of the respective electrocardiogram signal and to send the respective signal characteristic to the VT/SVT discrimination unit.

11. The cardiac stimulator according to one of Claims 1 to 10, **characterised in that** the morphology analysis unit is designed to determine a respective signal characteristic within a time window extending over a period of time which begins a predetermined amount of time before a respective detected ventricular cardiac activity and ends at a point in time that is after the detected ventricular cardiac activity by a predetermined amount of time.

12. The cardiac stimulator according to one of Claims 2 to 11, **characterised in that** the morphology analysis unit is connected to the ventricular sensing unit, which is designed to detect ventricular cardiac activity and to send a point in time of a detected ventricular cardiac activity to the morphology analysis unit.

## Revendications

1. Stimulateur cardiaque monoventriculaire (10) avec
- un boîtier (12) au moins partiellement électriquement conducteur,
- une première unité de détection (52) reliée ou destinée à être reliée à au moins une électrode ventriculaire et à une autre électrode, par le biais d'une ligne d'électrode ventriculaire, et conçue pour capter des actions cardiaques ventriculaires au moyen d'un premier signal d'électrocardiogramme, par le biais de l'électrode ventriculaire et de l'autre électrode,
- une deuxième unité de détection (70) reliée ou destinée à être reliée, d'une part à au moins une électrode de la ligne d'électrodes ventriculaire, par le biais de cette ligne d'électrodes ventriculaire, et d'autre part reliée à une autre électrode, et conçue pour capter un deuxième signal d'électrocardiogramme, par ces électrodes, simultanément avec le premier signal d'électrocardiogramme , dans lequel au moins l'une des deux électrodes par le biais desquelles le deuxième signal d'électrocardiogramme doit être capté est une autre électrode, par le biais de laquelle le premier signal d'électrocardiogramme doit être capté, de sorte que les signaux d'électrocardiogramme représentent des dérivations d'ECG dont les vecteurs sont différents de 0° ou de 180°,
- une unité d'analyse morphologique (76) reliée à la première et à la deuxième électrode et conçue pour déterminer respectivement au moins une caractéristique de signal morphologique du premier signal d'électrocardiogramme et du deuxième signal d'électrocardiogramme simultané, et
- une unité de discrimination VT/SVT (76) reliée à l'unité d'analyse morphologique ou comprenant celle-ci, et conçue pour utiliser les caractéristiques de signal déterminées par l'unité d'analyse morphologique pour la discrimination VT/SVT,
**caractérisé en ce que**
l'unité de discrimination VT/SVT est conçue pour déterminer le rapport entre les caractéristiques de signal déterminées par l'unité d'analyse morphologique et pour les comparer avec au moins une première valeur de rapport de référence.

2. Stimulateur cardiaque selon la revendication 1, **caractérisé en ce que** la première unité de détection est une unité de détection ventriculaire, reliée ou destinée à être reliée à au moins une paire d'électrodes de détection ventriculaire, par le biais d'une ligne d'électrodes ventriculaire, et conçue pour recevoir le premier signal d'électrocardiogramme par le biais de cette paire d'électrodes, en tant que signal d'électrocardiogramme bipolaire, et pour percevoir et classer (détecter) des actions cardiaques ventriculaires au moyen du signal d'électrocardiogramme reçu de façon bipolaire.

3. Stimulateur cardiaque selon la revendication 1 ou 2, **caractérisé en ce que** la deuxième unité de détection est reliée ou destinée à être reliée d'une part à au moins une électrode de défibrillation (bobine de choc) de la ligne d'électrodes ventriculaire par le biais de cette ligne d'électrodes ventriculaire, et d'autre part reliée au boîtier électriquement conducteur (12) du stimulateur cardiaque monoventriculaire, en tant qu'électrode supplémentaire, et conçue pour recevoir le deuxième signal d'électrocardiogramme en tant que signal d'électrocardiogramme de champ lointain, par le biais de ces deux électrodes.

4. Stimulateur cardiaque selon la revendication 1 ou 2, **caractérisé en ce que** la deuxième unité de détection est reliée ou destinée à être reliée à deux électrodes de défibrillation (bobines de choc) de la ligne d'électrodes ventriculaire par le biais de cette ligne d'électrodes ventriculaire, et conçue pour recevoir le deuxième signal d'électrocardiogramme en tant que signal d'électrocardiogramme de champ lointain par le biais de ces deux électrodes de défibrillation.

5. Stimulateur cardiaque selon l'une des revendications 1 à 4, **caractérisé en ce que** l'unité de discrimination VT/SVT est conçue pour comparer le rapport entre les caractéristiques de signal attribuées les unes aux autres et déterminées par l'unité d'analyse morphologique et la plage de tolérance autour de la première valeur de rapport de référence, et pour détecter une tachycardie ventriculaire (VT) lorsque la plage de tolérance est dépassée et une tachycardie supraventriculaire (SVT) lorsque la plage de tolérance n'est pas atteinte, ou inversement.

6. Stimulateur cardiaque selon la revendication 5, **caractérisé en ce que**
- le stimulateur cardiaque comporte une unité de chronométrage (345) conçue pour déterminer l'intervalle de temps entre des actions cardiaques ventriculaires consécutives, en tant qu'intervalle RR, ou sa réciproque en tant que rythme ventriculaire, et pour délivrer la valeur ainsi déterminée à l'unité de discrimination VT/SVT (76), et
- **en ce que** l'unité de discrimination VT/SVT (76) est conçue pour comparer un intervalle RR respectif ou un rythme ventriculaire respectif avec au moins une valeur seuil de zone VT, et pour déterminer, dans le cas où un intervalle RR serait inférieur à la valeur seuil de zone VT correspondante ou dans celui où un rythme ventriculaire respectif serait supérieur à la valeur seuil de zone VT correspondante, un rapport respectif entre les caractéristiques de signal déterminées par l'unité d'analyse morphologique, et pour comparer celui-ci avec au moins la première valeur de rapport de référence.

7. Stimulateur cardiaque selon la revendication 6, **caractérisé en ce que** l'unité de discrimination VT/SVT (76) est conçue pour stocker un rapport entre les caractéristiques de signal déterminées par l'unité d'analyse morphologique, en tant que valeur de rapport de référence, dans le cas où un intervalle RR respectivement correspondant serait plus long que la valeur seuil de zone VT ou dans celui où un rythme cardiaque ventriculaire respectivement correspondant serait inférieur à la valeur seuil de zone VT.

8. Stimulateur cardiaque selon la revendication 6 ou 7, **caractérisé en ce que** l'unité de chronométrage (345) est conçue pour calculer la moyenne d'un intervalle RR respectif ou de sa réciproque en tant que rythme ventriculaire respectif sur plusieurs cycles cardiaques consécutifs, et pour délivrer un intervalle RR respectif ainsi déterminé ou sa réciproque en tant que rythme ventriculaire respectif à l'unité de discrimination VT/SVT (76).

9. Stimulateur cardiaque selon l'une des revendications 5 à 8, **caractérisé en ce que** l'unité de discrimination VT/SVT (76) est conçue pour comparer un intervalle RR respectif ou un rythme ventriculaire respectif avec au moins deux valeurs seuil de zone VT, et pour attribuer l'intervalle RR respectif ou le rythme ventriculaire respectif à une zone VT, lorsque l'intervalle RR respectif est inférieur à une première valeur seuil de zone VT et supérieur à une deuxième valeur seuil de zone VT, ou lorsque le rythme ventriculaire respectif est supérieur à une première valeur seuil de zone VT et inférieur à une deuxième valeur seuil de zone VT, et pour attribuer l'intervalle RR respectif ou le rythme ventriculaire respectif à une zone VF lorsque l'intervalle RR respectif est inférieur à la première et à la deuxième valeur seuil de zone VT, ou lorsque le rythme ventriculaire respectif est supérieur à la première et à la deuxième valeur seuil de zone VT.

10. Stimulateur cardiaque selon l'une des revendications 1 à 9, **caractérisé en ce que** l'unité d'analyse morphologique est conçue pour déterminer une caractéristique de signal respective, y compris le signe correspondant de l'amplitude de signal du signal d'électrocardiogramme respectif, et pour délivrer celle-ci à l'unité de discrimination VT/SVT.

11. Stimulateur cardiaque selon l'une des revendications 1 à 10, **caractérisé en ce que** l'unité d'analyse morphologique est conçue pour déterminer une caractéristique de signal respective dans une fenêtre de temps s'étendant sur une durée débutant un certain temps avant une action cardiaque ventriculaire détectée et finissant à un moment autour d'un laps de temps prédéterminé après l'action cardiaque ventriculaire détectée.

12. Stimulateur cardiaque selon l'une des revendications 2 à 11, **caractérisé en ce que** l'unité d'analyse morphologique est reliée à l'unité de détection ventriculaire, laquelle est conçue pour détecter des actions cardiaques ventriculaires et pour indiquer à l'unité d'analyse morphologique le moment d'une action cardiaque ventriculaire détectée.
